# EUROPÄISCHE PATENTSCHRIFT

(11) **EP 2 944 640 B1**
(45) Veröffentlichungstag und Bekanntmachung des Hinweises auf die Patenterteilung: **28.09.2016**
(21) Anmeldenummer: 15161560.6
(22) Anmeldetag: 30.03.2015
(51) Int. Cl.: C07F 7/18

(54) **Harnstoffhaltige Silane, Verfahren zu deren Herstellung und deren Verwendung**
Urea-containing silanes, process for preparation thereof and use thereof
Silanes contenant de l'urée, leur procédé de fabrication et d'utilisation

(30) Priorität: 15.05.2014 DE 102014209226
(43) Veröffentlichungstag der Anmeldung: 18.11.2015
(73) Patentinhaber: Evonik Degussa GmbH, 45128 Essen (DE)
(72) Erfinder: Röben, Caren, 50670 Köln (DE); Moser, Ralph, 79104 Freiburg i. Br. (DE); Forster, Frank, 63825 Schöllkrippen (DE)

(56) Entgegenhaltungen:
- EP-A2- 1 156 053
- DE-A1- 2 360 471
- JP-A- 2002 201 312
- US-A1- 2003 191 270
- ERIC BESSON ET AL: "Soft route for monodisperse gold nanoparticles confined within SH-functionalized walls of mesoporous silica", JOURNAL OF MATERIALS CHEMISTRY, Bd. 19, Nr. 27, 1. Januar 2009 (2009-01-01), Seite 4746, XP055213535, ISSN: 0959-9428, DOI: 10.1039/b902568e

## Beschreibung

Die Erfindung betrifft harnstoffhaltige Silane, Verfahren zur deren Herstellung sowie deren Verwendung.

Aus CAS 1184961-62-3, 442527-46-0 und 498553-03-0 sind Verbindungen der Formel 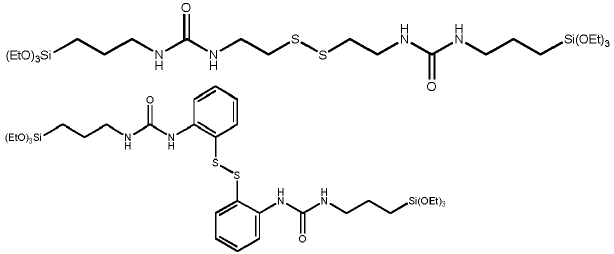 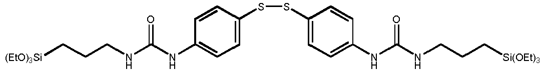 bekannt.

Ferner sind aus US 20030191270 A1 Silane der Formel 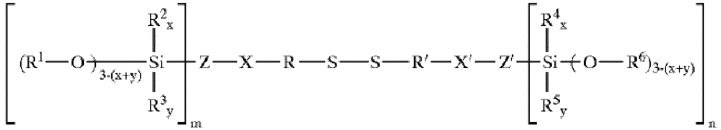 bekannt.

Aus JP 2002201312 A sind Kautschukmodifizierungsmittel der Formel 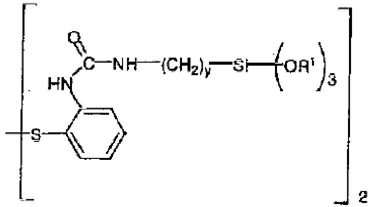 bekannt.

Ferner sind aus J. Mat. Chem. 2009, 19, 4746-4752 Goldnanopartikel innerhalb SHfunktionalisierter Gerüststrukturen aus mesoporösen Kieselsäuren und die Herstellung von harnstoffhaltigen Silanen bekannt. Bei dem bekannten Verfahren werden organische Lösungsmittel eingesetzt.

Nachteile der bekannten harnstoffhaltigen Disulfidsilane sind das schlechte Verstärkungsverhalten und der hohe Rollwiderstand.

Aufgabe der vorliegenden Erfindung ist es, harnstoffhaltige Silane zur Verfügung zu stellen, welche gegenüber aus dem Stand der Technik bekannten harnstoffhaltigen Silanen ein verbessertes Verstärkungsverhalten und Rollwiderstand in Kautschukmischungen aufweisen.

Gegenstand der Erfindung ist ein harnstoffhaltiges Silan der Formel I 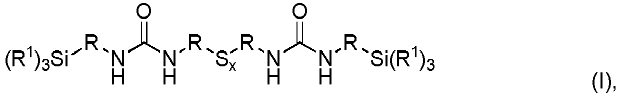 wobei R¹ gleich oder verschieden sind und C1-C10-Alkoxygruppen, vorzugsweise Methoxy- oder Ethoxygruppe, C2-C10- cyklische Dialkoxy-Gruppen, Phenoxygruppe, C4-C10-Cycloalkoxygruppen, C6-C20-Arylgruppen, vorzugsweise Phenyl, C1-C10-Alkylgruppen, vorzugsweise Methyl oder Ethyl, C2-C20-Alkenylgruppe, C7-C20-Aralkylgruppe oder Halogen, vorzugsweise Cl, und R gleich oder verschieden sind und eine verzweigte oder unverzweigte, gesättigte oder ungesättigte, aliphatische, aromatische oder gemischt aliphatische/aromatische zweibindige C1-C30-, bevorzugt C1-C20-, besonders bevorzugt C1-C10-, ganz besonders bevorzugt C1-C7-, insbesondere bevorzugt C2 und C3-Kohlenwasserstoffgruppe, die gegebenenfalls mit F-, Cl-, Br-, I-, -CN oder HS- substituiert ist, sind und x eine ganze Zahl von 3 bis 8, vorzugsweise 3 oder 4, ist.

Harnstoffhaltige Silane können Mischungen von harnstoffhaltigen Silanen der Formel I sein.

Das Verfahrensprodukt kann Oligomere, die durch Hydrolyse und Kondensation der Alkoxysilanfunktionen der harnstoffhaltigen Silanen der Formel I entstehen, enthalten.

Die harnstoffhaltigen Silane der Formel I können auf einem Träger, beispielsweise Wachs, Polymer oder Ruß, aufgebracht sein. Die harnstoffhaltigen Silane der Formel I können auf einer Kieselsäure aufgebracht sein, wobei die Anbindung physikalisch oder chemisch erfolgt sein kann.

R kann bevorzugt -CH₂-, -CH₂CH₂-, -CH₂CH₂CH₂-, -CH₂CH₂CH₂CH₂-, -CH(CH₃)-, -CH₂CH(CH₃)-, - CH(CH₃)CH₂-, -C(CH₃)₂-, -CH(C₂H₅)-, -CH₂CH₂CH(CH₃)-, -CH(CH₃)CH₂CH₂-,

-CH₂CH(CH₃)CH₂-, -CH₂CH₂CH₂CH₂CH₂-, -CH₂CH₂CH₂CH₂CH₂CH₂-,
-CH₂CH₂CH₂CH₂CH₂CH₂CH₂-, -CH₂CH₂CH₂CH₂CH₂CH₂CH₂CH₂-,
-CH₂CH₂CH₂CH₂CH₂CH₂CH₂CH₂CH₂-, -CH₂CH₂CH₂CH₂CH₂CH₂CH₂CH₂CH₂CH₂-,
-CH₂CH₂CH₂CH₂CH₂CH₂CH₂CH₂CH₂CH₂CH₂CH₂-,
-CH₂CH₂CH₂CH₂CH₂CH₂CH₂CH₂CH₂CH₂CH₂CH₂CH₂CH₂-,
-CH₂CH₂CH₂CH₂CH₂CH₂CH₂CH₂CH₂CH₂CH₂CH₂CH₂CH₂CH₂CH₂-,
-CH₂CH₂CH₂CH₂CH₂CH₂CH₂CH₂CH₂CH₂CH₂CH₂CH₂CH₂CH₂CH₂CH₂CH₂-oder 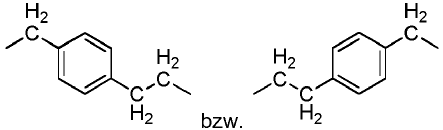 bedeuten. R¹ kann bevorzugt Methoxy oder Ethoxy sein.

Harnstoffhaltige Silane der Formel I können bevorzugt sein:
((EtO)₃Si-CH₂-NH-CO-NH-CH₂-S_{x/2})₂,
((EtO)₃Si-CH₂CH₂-NH-CO-NH-CH₂-S_{x/2})₂,
((EtO)₃Si-CH₂-NH-CO-NH-CH₂CH₂-S_{x/2})₂,
((EtO)₃Si-CH₂CH₂-NH-CO-NH-CH₂CH₂-S_{x/2})₂,
((EtO)₃Si-CH₂CH₂CH₂-NH-CO-NH-CH₂-S_{x/2})₂,
((EtO)₃Si-CH₂CH₂CH₂-NH-CO-NH-CH₂CH₂-S_{x/2})₂,
((EtO)₃Si-CH₂-NH-CO-NH-CH₂CH₂CH₂-S_{x/2})₂,
((EtO)₃Si-CH₂CH₂-NH-CO-NH-CH₂CH₂CH₂-S_{x/2})₂,
((EtO)₃Si-CH₂CH₂CH₂-NH-CO-NH-CH₂CH₂CH₂-S_{x/2})₂,
((MeO)₃Si-CH₂-NH-CO-NH-CH₂-S_{x/2})₂,
((MeO)₃Si-CH₂CH₂-NH-CO-NH-CH₂-S_{x/2})₂,
((MeO)₃Si-CH₂-NH-CO-NH-CH₂CH₂-S_{x/2})₂,
((MeO)₃Si-CH₂CH₂-NH-CO-NH-CH₂CH₂-S_{x/2})₂,
((MeO)₃Si-CH₂CH₂CH₂-NH-CO-NH-CH₂-S_{x/2})₂,
((MeO)₃Si-CH₂CH₂CH₂-NH-CO-NH-CH₂CH₂-S_{x/2})₂,
((MeO)₃Si-CH₂-NH-CO-NH-CH₂CH₂CH₂-S_{x/2})₂,
((MeO)₃Si-CH₂CH₂-NH-CO-NH-CH₂CH₂CH₂-S_{x/2})₂, oder
((MeO)₃Si-CH₂CH₂CH₂-NH-CO-NH-CH₂CH₂CH₂-S_{x/2})₂.

Insbesondere bevorzugt ist die Verbindung der Formel (EtO)₃Si-CH₂CH₂CH₂-NH-CO-NH-CH₂CH₂-S₄-CH₂CH₂-NH-CO-NH-CH₂CH₂CH₂-Si(OEt)₃.

Ein weiterer Gegenstand der Erfindung ist ein erstes Verfahren zur Herstellung der erfindungsgemäßen harnstoffhaltigen Silane der Formel I 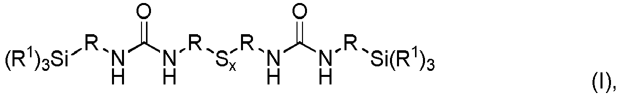 wobei R¹, R und x die oben genannte Bedeutung haben, welches dadurch gekennzeichnet ist, dass man ein harnstoffhaltiges Disulfidsilane der Formel II 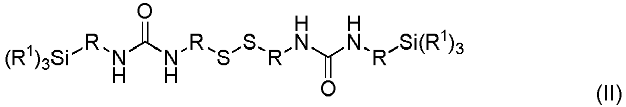 mit Schwefel umsetzt.

Die Reaktion kann unter Luftausschluss durchgeführt werden.

Die Reaktion kann unter Schutzgasatmosphäre durchgeführt werden, zum Beispiel unter Argon oder Stickstoff, bevorzugt unter Stickstoff.

Das erste erfindungsgemäße Verfahren kann bei Normaldruck, erhöhtem Druck oder reduziertem Druck durchgeführt werden. Bevorzugt kann das erfindungsgemäße Verfahren bei Normaldruck durchgeführt werden.

Erhöhter Druck kann eine Druck von 1,1 bar bis 100 bar, bevorzugt von 1,5 bar bis 50 bar, besonders bevorzugt von 2 bar bis 20 bar und ganz besonders bevorzugt von 2 bis 10 bar, sein.

Reduzierter Druck kann ein Druck von 1 mbar bis 1000 mbar, bevorzugt 1 mbar bis 500 mbar, besonders bevorzugt 1 mbar bis 250 mbar, ganz besonders bevorzugt 5 mbar bis 100 mbar, sein.

Das erste erfindungsgemäße Verfahren kann zwischen 30°C und 180°C, bevorzugt zwischen 80°C und 165°C, besonders bevorzugt zwischen 120°C und 160°C durchgeführt werden.

Die Umsetzung kann in einem Lösungsmittel, beispielsweise Methanol, Ethanol, Propanol, Butanol, Cyclohexanol, N,N-Dimethylformamid, Dimethylsulfoxid, Pentan, Hexan, Cyclohexan, Heptan, Octan, Dekan, Toluol, Xylol, Aceton, Acetonitril, Tetrachlorkohlenstoff, Chloroform, Dichlormethan, 1,2-Dichlormethan, Tetrachlorethylen, Diethylether, Methyltertbutylether, Methylethylketon, Tetrahydrofuran, Dioxan, Pyridin oder Essigsäuremethylester, erfolgen. Die Umsetzung kann bevorzugt ohne Lösungsmittel erfolgen.

Die Umsetzung kann ohne organische Lösungsmittel durchgeführt werden. Das Lösungsmittel kann Wasser sein.

Das durch das erste erfindungsgemäße Verfahren erhältliche harnstoffhaltige Silan der Formel I kann in einer Ausbeute von größer als 50%, bevorzugt größer als 60%, ganz besonders bevorzugt größer als 70%, erhalten werden.

Ein weiterer Gegenstand der Erfindung ist ein zweites Verfahren zur Herstellung der erfindungsgemäßen harnstoffhaltigen Silane der Formel I 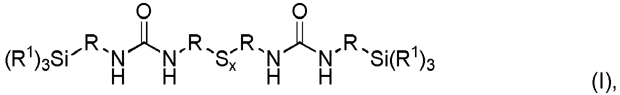 wobei R¹, R und x die oben genannte Bedeutung haben, welches dadurch gekennzeichnet ist, dass man in einem ersten Schritt ein Aminosilan der Formel III 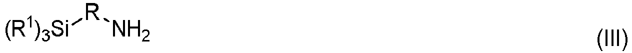 mit einem Isocyanat der Formel IV 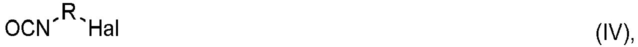 wobei R und R¹ die oben genannte Bedeutungen haben und Hal gleich F, Cl, Br oder I, vorzugsweise Cl, ist,
umsetzt
und in einem zweiten Schritt das Produkt aus dem ersten Verfahrensschritt mit Natriumpolysulfid der Formel (V)

Na₂Sₓ (V)

wobei x die oben genannte Bedeutung hat, umsetzt.

Aminosilane der Formel III können bevorzugt sein:
(C₂H₅O)₃Si-CH₂,
(C₂H₅O)₃Si-CH₂CH₂-NH₂,
(C₂H₅O)₃Si-CH₂CH₂CH₂-NH₂,
(CH₃O)₃Si-CH₂-NH₂,
(CH₃O)₃Si-CH₂CH₂-NH₂ oder
(CH₃O)₃Si-CH₂CH₂CH₂-NH₂.

Isocyanate der Formel IV können bevorzugt sein:
OCN-CH₂-Cl,
OCN-CH₂CH₂-Cl oder
OCN-CH₂CH₂CH₂-Cl.

Bei dem zweiten erfindungsgemäßen Verfahren kann der erste und zweite Verfahrensschritt in einem Reaktionsbehälter durch Zugabe aller Edukte erfolgen.

Bei dem ersten Schritt des zweiten erfindungsgemäßen Verfahrens kann Aminosilan der Formel III zu Isocyanat der Formel IV dosiert werden.

Bei dem ersten Schritt des zweiten erfindungsgemäßen Verfahrens kann bevorzugt das Isocyanat der Formel IV zu Aminosilan der Formel III dosiert werden.

Bei dem ersten Schritt des zweiten erfindungsgemäßen Verfahrens kann das Aminosilan der Formel III zu Isocyanat der Formel IV im molekularen Verhältnis von 0,85:1 bis 1,15:1, bevorzugt 0,90:1 bis 1,10:1, besonders bevorzugt im Verhältnis 0,95:1 bis 1,05:1, eingesetzt werden.

Die Reaktion im ersten Schritt des zweiten erfindungsgemäßen Verfahrens kann unter Luftausschluss durchgeführt werden.

Die Reaktion im ersten Schritt des zweiten erfindungsgemäßen Verfahrens kann unter Schutzgasatmosphäre durchgeführt werden, zum Beispiel unter Argon oder Stickstoff, bevorzugt unter Stickstoff.

Der erste Schritt des erfindungsgemäßen zweiten Verfahrens kann bei Normaldruck, erhöhtem Druck oder reduziertem Druck durchgeführt werden. Bevorzugt kann das erfindungsgemäße Verfahren bei Normaldruck durchgeführt werden.

Erhöhter Druck kann ein Druck von 1,1 bar bis 100 bar, bevorzugt von 1,5 bar bis 50 bar, besonders bevorzugt von 2 bar bis 20 bar und ganz besonders bevorzugt von 2 bis 10 bar, sein.

Reduzierter Druck kann ein Druck von 1 mbar bis 1000 mbar, bevorzugt 1 mbar bis 500 mbar, besonders bevorzugt 1 mbar bis 250 mbar, ganz besonders bevorzugt 5 mbar bis 100 mbar, sein.

Der erste Schritt des zweiten erfindungsgemäßen Verfahrens kann zwischen -78°C und 100°C, bevorzugt zwischen -70°C und 50°C, besonders bevorzugt zwischen -65°C und 25°C durchgeführt werden.

Die Umsetzung im ersten Schritt des zweiten erfindungsgemäßen Verfahrens kann ohne Lösungsmittel oder in einem Lösungsmittel, beispielsweise Methanol, Ethanol, Propanol, Butanol, Cyclohexanol, N,N-Dimethylformamid, Dimethylsulfoxid, Pentan, Hexan, Cyclohexan, Heptan, Octan, Dekan, Toluol, Xylol, Aceton, Acetonitril, Tetrachlorkohlenstoff, Chloroform, Dichlormethan, 1,2-Dichlormethan, Tetrachlorethylen, Diethylether, Methyltertbutylether, Methylethylketon, Tetrahydrofuran, Dioxan, Pyridin oder Ethylacetat, erfolgen. Das Lösungsmittel kann bevorzugt Dichlormethan, Ethanol, Methyltertbutylether, Toluol, Ethylacetat, Pentan, oder Hexan sein.

Die Umsetzung im ersten Schritt des zweiten erfindungsgemäßen Verfahrens kann ohne organisches Lösungsmitteln durchgeführt werden. Das Lösungsmittel kann Wasser sein.

Das Lösungsmittel im ersten Schritt des zweiten erfindungsgemäßen Verfahrens kann anschließend entfernt, vorzugsweise abdestilliert, werden.

Das Reaktionsprodukt aus dem ersten Schritt des zweiten erfindungsgemäßen Verfahrens kann anschließend filtriert werden und mit einem organischen Lösungsmittel gewaschen werden. Bevorzugt kann mit einem Alkan, besonders bevorzugt Hexan, gewaschen werden.

Das Reaktionsprodukt aus dem ersten Schritt des zweiten erfindungsgemäßen Verfahrens kann nach der Filtration getrocknet werden. Die Trocknung kann bei Temperaturen von 20°C - 100°C, vorzugsweise von 25°C - 50°C, erfolgen. Die Trocknung kann bei Unterdruck von 1 - 500 mbar erfolgen.

Das durch das zweite erfindungsgemäße Verfahren im ersten Schritt erhältliche harnstoffhaltige Halogensilan der Formel VI 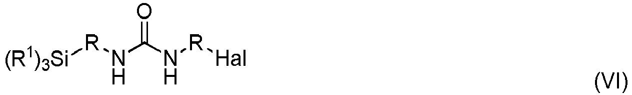 kann in einer Ausbeute von größer als 50%, bevorzugt größer als 60%, ganz besonders bevorzugt größer als 70%, erhalten werden.

Die Reaktion im zweiten Schritt des zweiten erfindungsgemäßen Verfahrens kann unter Luftausschluss durchgeführt werden.

Die Reaktion im zweiten Schritt des zweiten erfindungsgemäßen Verfahrens kann unter Schutzgasatmosphäre durchgeführt werden, zum Beispiel unter Argon oder Stickstoff, bevorzugt unter Stickstoff.

Der zweite Schritt des zweiten erfindungsgemäßen Verfahrens kann bei Normaldruck, erhöhtem Druck oder reduziertem Druck durchgeführt werden. Bevorzugt kann das erfindungsgemäße Verfahren bei Normaldruck durchgeführt werden.

Erhöhter Druck kann ein Druck von 1,1 bar bis 100 bar, bevorzugt von 1,5 bar bis 50 bar, besonders bevorzugt von 2 bar bis 20 bar und ganz besonders bevorzugt von 2 bis 10 bar, sein.

Reduzierter Druck kann ein Druck von 1 mbar bis 1000 mbar, bevorzugt 1 mbar bis 500 mbar, besonders bevorzugt 1 mbar bis 250 mbar, ganz besonders bevorzugt 5 mbar bis 100 mbar, sein.

Der zweite Schritt des zweiten erfindungsgemäßen Verfahrens kann zwischen 20°C und 150°C, bevorzugt zwischen 40°C und 100°C, besonders bevorzugt zwischen 45°C und 80°C durchgeführt werden.

Die Umsetzung im zweiten Schritt des zweiten erfindungsgemäßen Verfahrens kann ohne Lösungsmittel oder in einem Lösungsmittel, beispielsweise Methanol, Ethanol, Propanol, Butanol, Cyclohexanol, N,N-Dimethylformamid, Dimethylsulfoxid, Pentan, Hexan, Cyclohexan, Heptan, Octan, Dekan, Toluol, Xylol, Aceton, Acetonitril, Tetrachlorkohlenstoff, Chloroform, Dichlormethan, 1,2-Dichlormethan, Tetrachlorethylen, Diethylether, Methyltertbutylether, Methylethylketon, Tetrahydrofuran, Dioxan, Pyridin oder Ethylacetat, erfolgen. Das Lösungsmittel kann bevorzugt Ethanol sein.

Die Umsetzung im zweiten Schritt des zweiten erfindungsgemäßen Verfahrens kann ohne organisches Lösungsmitteln durchgeführt werden. Das Lösungsmittel kann Wasser sein.
Das Reaktionsprodukt im zweiten Schritt des zweiten erfindungsgemäßen Verfahrens kann filtriert werden und der Filterkuchen mit einem organischen Lösungsmittel gewaschen werden. Bevorzugt kann mit einem Alkohol, besonders bevorzugt Ethanol, oder einem Alkan, besonders bevorzugt Hexan, gewaschen werden.

Das Lösungsmittel im zweiten Schritt des zweiten erfindungsgemäßen Verfahrens kann anschließend entfernt, vorzugsweise abdestilliert, werden.

Das Reaktionsprodukt im zweiten Schritt des zweiten erfindungsgemäßen Verfahrens kann nach der Filtration und Lösungsmittelentfernung getrocknet werden. Die Trocknung kann bei Temperaturen von 20°C - 100°C, vorzugsweise von 25°C - 50°C, erfolgen. Die Trocknung kann bei Unterdruck von 1 - 500 mbar erfolgen.

Das durch das zweite erfindungsgemäße Verfahren im zweiten Schritt erhältliche harnstoffhaltige Silan der Formel I 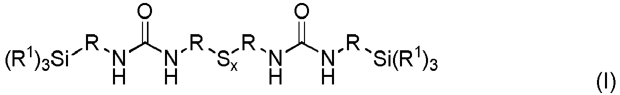 kann in einer Ausbeute von größer als 50%, bevorzugt größer als 60%, ganz besonders bevorzugt größer als 70%, erhalten werden.

In einer bevorzugten Ausführungsform des zweiten erfindungsgemäßen Verfahrens kann bei -78 bis -50°C das Isocyanat der Formel IV zu dem Aminosilan der Formel III in Ethanol dosiert, die Reaktionsmischung danach auf 50°C erwärmt, Natriumpolysulfid der Formel V portionsweise zugegeben, danach refluxiert, vorzugsweise bei 78 °C, nach beendeter Reaktion abgekühlt, filtriert und das Lösungsmittel Ethanol unter Vakuum entfernt werden.

Das Produkt, hergestellt mit dem zweiten erfindungsgemäßen Verfahren, kann einen Restgehalt an harnstoffhaltigem Halogensilan der Formel VI von weniger als 25 mol-%, bevorzugt weniger als 10 mol-%, besonders bevorzugt weniger als 5 mol-%, ganz besonders bevorzugt weniger als 3 mol-%, haben.

Die relativen mol-% der harnstoffhaltigen Halogensilane der Formel VI in dem Produkt, hergestellt mit dem zweiten erfindungsgemäßen Verfahren, werden im ¹H-NMR durch Integration der H-Atome der -CH₂CH₂-Cl Gruppe der Verbindungen der Formel VI gegen die H-Atome der Si-CH₂- Gruppe des harnstoffhaltigen Silans der Formel I bestimmt.
Für die Substanz der Formel VI (EtO)₃Si-CH₂CH₂CH₂-NH-CO-NH-CH₂CH₂-Cl wird zum Beispiel das Integral der H-Atome der -CH₂CH₂-Cl Gruppe (δ = 3.17 ppm) für die Bestimmung der relativen Gehalte verwendet.

Das Produkt, hergestellt mit dem zweiten erfindungsgemäßen Verfahren, kann einen Restgehalt an Aminosilan der Formel III von weniger als 10 mol-%, bevorzugt weniger als 5 mol-%, besonders bevorzugt weniger als 1 mol-%, ganz besonders bevorzugt weniger als 0,1 mol-%, haben.

Die relativen mol-% der Aminosilane der Formel III in dem Produkt, hergestellt mit dem zweiten erfindungsgemäßen Verfahren, werden im ¹³C-NMR durch Integration der C-Atome der -CH₂-NH₂ Gruppe der Aminosilane der Formel III gegen die C-Atome der Si-CH₂- Gruppe des harnstoffhaltigen Silans der Formel I bestimmt.
Für die Substanz der Formel III (EtO)₃Si-CH₂-CH₂-CH₂-NH₂ wird zum Beispiel das Integral der C - Atome der -CH₂-NH₂ Gruppe (δ = 45,15 ppm) für die Bestimmung der relativen Gehalte verwendet.

Das Produkt, hergestellt mit dem zweiten erfindungsgemäßen Verfahren, kann einen Restgehalt an Isocyanat der Formel IV von weniger als 25 mol-%, bevorzugt weniger als 10 mol-%, besonders bevorzugt weniger als 5 mol-%, ganz besonders bevorzugt weniger als 3 mol-%, haben.

Die relativen mol-% der Isocyanate der Formel IV in dem Produkt, hergestellt mit dem zweiten erfindungsgemäßen Verfahren, werden im ¹³C-NMR durch Integration der C-Atome der OCN-CH₂-Gruppe der Isocyanate der Formel IV gegen die C-Atome der Si-CH₂- Gruppe des harnstoffhaltigen Silans der Formel I bestimmt.
Für die Substanz der Formel IV OCN-CH₂-CH₂-Cl wird zum Beispiel das Integral der C-Atome der OCN-CH₂- Gruppe (δ = 124,33 ppm) für die Bestimmung der relativen Gehalte verwendet.
Ein weiterer Gegenstand der Erfindung ist ein drittes Verfahren zur Herstellung der erfindungsgemäßen harnstoffhaltigen Silane der Formel I 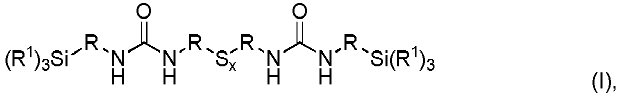 wobei R¹, R und x die oben genannte Bedeutung haben, welches dadurch gekennzeichnet ist, dass man in einem ersten Schritt ein Isocyanatosilan der Formel VII 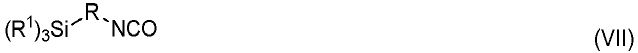 mit einem Amin der Formel VIII 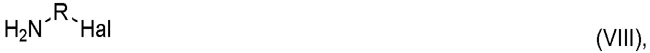 wobei R und R¹ die oben genannte Bedeutungen haben und Hal gleich F, Cl, Br oder I, vorzugsweise Cl, ist, umsetzt
und in einem zweiten Schritt das Produkt aus dem ersten Verfahrensschritt mit Natriumpolysulfid der Formel (V)

Na₂Sₓ (V)

wobei x die oben genannte Bedeutung hat,
umsetzt.

Isocyanatosilane der Formel VII können bevorzugt sein:
(C₂H₅O)₃Si-CH₂-NCO,
(C₂H₅O)₃Si-CH₂CH₂-NCO,
(C₂H₅O)₃Si-CH₂CH₂CH₂-NCO,
(CH₃O)₃Si-CH₂-NCO,
(CH₃O)₃Si-CH₂CH₂-NCO oder
(CH₃O)₃Si-CH₂CH₂CH₂-NCO.

Amine der Formel VIII können bevorzugt sein:
H₂N-CH₂-Cl,
H₂N-CH₂CH₂-Cl oder
H₂N-CH₂CH₂CH₂-Cl.

Bei dem dritten erfindungsgemäßen Verfahren kann der erste und zweite Verfahrensschritt in einem Reaktionsbehälter durch Zugabe aller Edukte erfolgen.

Bei dem ersten Schritt des dritten erfindungsgemäßen Verfahrens kann Amin der Formel VIII zu Isocyanatosilan der Formel VII dosiert werden.

Bei dem ersten Schritt des dritten erfindungsgemäßen Verfahrens kann bevorzugt das Isocyanatosilan der Formel VII zu Aminen der Formel VIII dosiert werden.

Bei dem ersten Schritt des dritten erfindungsgemäßen Verfahrens kann das Isocyanatosilan der Formel VII zu Amin der Formel VIII im molekularen Verhältnis von 0,85:1 bis 1,15:1, bevorzugt 0,90:1 bis 1,10:1, besonders bevorzugt im Verhältnis 0,95:1 bis 1,05:1, eingesetzt werden.
Die Reaktion im ersten Schritt des dritten erfindungsgemäßen Verfahrens kann unter Luftausschluss durchgeführt werden.

Die Reaktion im ersten Schritt des dritten erfindungsgemäßen Verfahrens kann unter Schutzgasatmosphäre durchgeführt werden, zum Beispiel unter Argon oder Stickstoff, bevorzugt unter Stickstoff.

Der erste Schritt des dritten erfindungsgemäßen Verfahrens kann bei Normaldruck, erhöhtem Druck oder reduziertem Druck durchgeführt werden. Bevorzugt kann das erfindungsgemäße Verfahren bei Normaldruck durchgeführt werden.

Erhöhter Druck kann ein Druck von 1,1 bar bis 100 bar, bevorzugt von 1,5 bar bis 50 bar, besonders bevorzugt von 2 bar bis 20 bar und ganz besonders bevorzugt von 2 bis 10 bar, sein.

Reduzierter Druck kann ein Druck von 1 mbar bis 1000 mbar, bevorzugt 1 mbar bis 500 mbar, besonders bevorzugt 1 mbar bis 250 mbar, ganz besonders bevorzugt 5 mbar bis 100 mbar, sein. Der erste Schritt des dritten erfindungsgemäßen Verfahrens kann zwischen -78°C und 100°C, bevorzugt zwischen -75°C und 60°C, besonders bevorzugt zwischen -70°C und 40°C durchgeführt werden.

Die Umsetzung im ersten Schritt des dritten erfindungsgemäßen Verfahrens kann ohne Lösungsmittel oder in einem Lösungsmittel, beispielsweise Methanol, Ethanol, Propanol, Butanol, Cyclohexanol, N,N-Dimethylformamid, Dimethylsulfoxid, Pentan, Hexan, Cyclohexan, Heptan, Octan, Dekan, Toluol, Xylol, Aceton, Acetonitril, Tetrachlorkohlenstoff, Chloroform, Dichlormethan, 1,2-Dichlormethan, Tetrachlorethylen, Diethylether, Methyltertbutylether, Methylethylketon, Tetrahydrofuran, Dioxan, Pyridin oder Ethylacetat, erfolgen.

Die Umsetzung im ersten Schritt des dritten erfindungsgemäßen Verfahrens kann ohne organisches Lösungsmitteln durchgeführt werden. Das Lösungsmittel kann bevorzugt Ethanol sein.

Das Lösungsmittel im ersten Schritt des dritten erfindungsgemäßen Verfahrens kann anschließend entfernt, vorzugsweise abdestilliert, werden.

Das Reaktionsprodukt im ersten Schritt des dritten erfindungsgemäßen Verfahrens kann anschließend gefiltert werden und mit einem organischen Lösungsmittel gewaschen werden. Bevorzugt kann mit einem Alkan, besonders bevorzugt Hexan, gewaschen werden.

Das Reaktionsprodukt im ersten Schritt des dritten erfindungsgemäßen Verfahrens kann nach der Filterung getrocknet werden. Die Trocknung kann bei Temperaturen von 20°C - 100°C, vorzugsweise von 25°C - 50°C, erfolgen. Die Trocknung kann bei Unterdruck von 1 - 500 mbar erfolgen.

Das im ersten Schritt des dritten erfindungsgemäßen Verfahrens erhältliche harnstoffhaltige Halogensilan der Formel VI 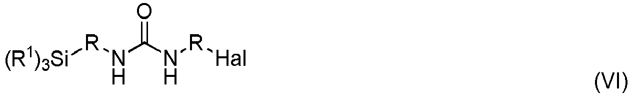 kann in einer Ausbeute von größer als 50%, bevorzugt größer als 60%, ganz besonders bevorzugt größer als 70%, erhalten werden.

Die Reaktion im zweiten Schritt des dritten erfindungsgemäßen Verfahrens kann unter Luftausschluss durchgeführt werden.

Die Reaktion im zweiten Schritt des dritten erfindungsgemäßen Verfahrens kann unter Schutzgasatmosphäre durchgeführt werden, zum Beispiel unter Argon oder Stickstoff, bevorzugt unter Stickstoff.

Der zweite Schritt des dritten erfindungsgemäßen Verfahrens kann bei Normaldruck, erhöhtem Druck oder reduziertem Druck durchgeführt werden. Bevorzugt kann das erfindungsgemäße Verfahren bei Normaldruck durchgeführt werden.

Erhöhter Druck kann ein Druck von 1,1 bar bis 100 bar, bevorzugt von 1,5 bar bis 50 bar, besonders bevorzugt von 2 bar bis 20 bar und ganz besonders bevorzugt von 2 bis 10 bar, sein.

Reduzierter Druck kann ein Druck von 1 mbar bis 1000 mbar, bevorzugt 1 mbar bis 500 mbar, besonders bevorzugt 1 mbar bis 250 mbar, ganz besonders bevorzugt 5 mbar bis 100 mbar, sein.

Der zweite Schritt des dritten erfindungsgemäßen Verfahrens kann zwischen 20°C und 150°C, bevorzugt zwischen 40°C und 100°C, besonders bevorzugt zwischen 45°C und 80°C durchgeführt werden.

Die Umsetzung im zweiten Schritt des dritten erfindungsgemäßen Verfahrens kann ohne Lösungsmittel oder in einem Lösungsmittel, beispielsweise Methanol, Ethanol, Propanol, Butanol, Cyclohexanol, N,N-Dimethylformamid, Dimethylsulfoxid, Pentan, Hexan, Cyclohexan, Heptan, Octan, Dekan, Toluol, Xylol, Aceton, Acetonitril, Tetrachlorkohlenstoff, Chloroform, Dichlormethan, 1,2-Dichlormethan, Tetrachlorethylen, Diethylether, Methyltertbutylether, Methylethylketon, Tetrahydrofuran, Dioxan, Pyridin oder Ethylacetat, erfolgen. Das Lösungsmittel kann bevorzugt Ethanol sein.

Die Umsetzung im zweiten Schritt des dritten erfindungsgemäßen Verfahrens kann ohne organisches Lösungsmitteln durchgeführt werden. Das Lösungsmittel kann Wasser sein.

Das Reaktionsprodukt aus dem zweiten Schritt des dritten erfindungsgemäßen Verfahrens kann filtriert werden und der Filterkuchen mit einem organischen Lösungsmittel gewaschen werden. Bevorzugt kann mit einem Alkohol, besonders bevorzugt Ethanol, oder einem Alkan, besonders bevorzugt Hexan, gewaschen werden.

Das Lösungsmittel im zweiten Schritt des dritten erfindungsgemäßen Verfahrens kann anschließend entfernt, vorzugsweise abdestilliert, werden.

Das Reaktionsprodukt im zweiten Schritt des dritten erfindungsgemäßen Verfahrens kann nach der Filtration und Lösungsmittelentfernung getrocknet werden. Die Trocknung kann bei Temperaturen von 20°C - 100°C, vorzugsweise von 25°C - 50°C, erfolgen. Die Trocknung kann bei Unterdruck von 1 - 500 mbar erfolgen.
Das im zweiten Schritt durch das dritte erfindungsgemäße Verfahren erhältliche harnstoffhaltige Silan der Formel I 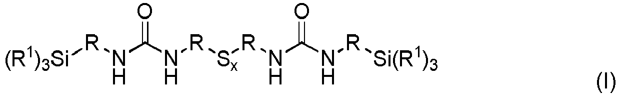 kann in einer Ausbeute von größer als 50%, bevorzugt größer als 60%, ganz besonders bevorzugt größer als 70%, erhalten werden.

In einer bevorzugten Ausführungsform des dritten erfindungsgemäßen Verfahrens kann bei -78 bis -50°C das Isocyanatosilan der Formel VII zu dem Amin der Formel VIII in Ethanol dosiert, die Reaktionsmischung danach auf 50°C erwärmt, Natriumpolysulfid der Formel V portionsweise zugegeben, danach refluxiert, vorzugsweise bei 78 °C, nach beendeter Reaktion abgekühlt, filtriert und das Lösungsmittel Ethanol unter Vakuum entfernt werden.

Das Produkt, hergestellt mit dem dritten erfindungsgemäßen Verfahren, kann einen Restgehalt an harnstoffhaltigem Halogensilan der Formel VI von weniger als 25 mol-%, bevorzugt weniger als 10 mol-%, besonders bevorzugt weniger als 5 mol-%, ganz besonders bevorzugt weniger als 3 mol-%, haben.

Die relativen mol-% der harnstoffhaltigen Halogensilane der Formel VI in dem Produkt, hergestellt mit dem dritten erfindungsgemäßen Verfahren, werden im ¹H-NMR durch Integration der H-Atome der -CH₂CH₂-Cl Gruppe der harnstoffhaltigen Halogensilane der Formel VI gegen die H-Atome der Si-CH₂- Gruppe des harnstoffhaltigen Silans der Formel I bestimmt.
Für die Substanz der Formel VI (EtO)₃Si-CH₂CH₂CH₂-NH-CO-NH-CH₂CH₂-Cl wird zum Beispiel das Integral der H-Atome der -CH₂CH₂-Cl Gruppe (δ = 3.17 ppm) für die Bestimmung der relativen Gehalte verwendet.

Das Produkt, hergestellt mit dem dritten erfindungsgemäßen Verfahren, kann einen Restgehalt an Isocyanatosilan der Formel VII von weniger als 10 mol-%, bevorzugt weniger als 5 mol-%, besonders bevorzugt weniger als 1 mol-%, ganz besonders bevorzugt weniger als 0,1 mol-%, haben.

Die relativen mol-% der Isocyanatosilane der Formel VII in dem Produkt in einem Bereich >1 mol-%, hergestellt mit dem dritten erfindungsgemäßen Verfahren, werden im ¹³C-NMR durch Integration der C-Atome der -NCO Gruppe der Isocyanatosilane der Formel VII gegen die C-Atome der Si-CH₂- Gruppe des harnstoffhaltigen Silans der Formel I bestimmt.
Für die Substanz der Formel VII (EtO)₃Si-CH₂-CH₂-CH₂-NCO wird zum Beispiel das Integral der C-Atome der -NCO Gruppe (δ = 122,22 ppm) für die Bestimmung der relativen Gehalte in einem Bereich >1 mol-% verwendet.
Die relativen mol-% der Isocyanatosilane der Formel VII in dem Produkt in einem Bereich <1 mol-%, hergestellt mit dem dritten erfindungsgemäßen Verfahren, werden durch eine dem Fachmann bekannte quantitative FT-IR-Spektroskopie bestimmt. Die Kalibrierung der Methode erfolgt durch Verwendung von Kalibrierlösungen geeigneter Konzentration (z.B. in C₂Cl₄). Für die Messung werden ca. 1 g Probe in eine 25 ml Rollkragenflasche eingewogen und 25 g C₂Cl₄ zugegeben. Die Probe wird auf der Schüttelmaschine 1 - 2 Stunden geschüttelt. Im Anschluss wird die untere flüssige Phase vorsichtig in eine 20 mm IR-Küvette dosiert und per FT-IR-Spektroskopie vermessen (4000-1200 cm⁻¹, Auflösung 2 cm⁻¹). Unter gleichen Bedingungen wird ein Spektrum des Lösungsmittels zur Subtraktion aufgenommen.
Für die Substanz der Formel VII (EtO)₃Si-CH₂-CH₂-CH₂-NCO wird zum Beispiel die Wellenlänge der Valenzschwingung der -NCO Gruppe bei 2270 cm⁻¹ für die Bestimmung der relativen Gehalte in einem Bereich <1 mol-% verwendet.

Das Produkt, hergestellt mit dem dritten erfindungsgemäßen Verfahren, kann einen Restgehalt an Amin der Formel VIII von weniger als 25 mol-%, bevorzugt weniger als 10 mol-%, besonders bevorzugt weniger als 5 mol-%, ganz besonders bevorzugt weniger als 3 mol-%, haben.

Die relativen mol-% der Amine der Formel VIII in dem Produkt, hergestellt mit dem dritten erfindungsgemäßen Verfahren, werden im ¹³C-NMR durch Integration der C-Atome der -CH₂-NH₂ Gruppe der Amine der Formel VIII gegen die C-Atome der Si-CH₂- Gruppe des harnstoffhaltigen Silans der Formel I bestimmt.
Für die Substanz der Formel VIII H₂N-CH₂-CH₂-Cl wird zum Beispiel das Integral der C-Atome der H₂N-CH₂-CH₂-Cl Gruppe (δ = 39,47 ppm) oder der H₂N-CH₂-CH₂-Cl Gruppe (δ = 37,95 ppm) für die Bestimmung der relativen Gehalte verwendet.

Das Amin der Formel VIII kann vor der Umsetzung mit dem Isocyanatsilan der Formel VII aus dem Hydrochlorid-Salz des Amins der Formel IX 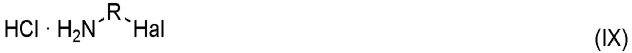 durch Zugabe einer Base, vorzugsweise NaOEt, hergestellt werden. Dabei kann die Base zugegeben werden bis sich ein pH-Wert zwischen 7 und 14 einstellt.

In einer bevorzugten Ausführungsform kann das dritte Verfahren zur Herstellung von harnstoffhaltigen Silanen der Formel I 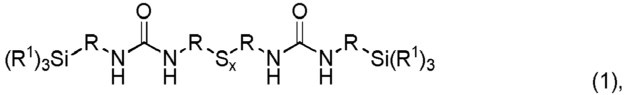 wobei x, R und R1 die oben genannte Bedeutung haben, dadurch gekennzeichnet sein, dass man das Hydrochloridsalz des Amins der Formel IX 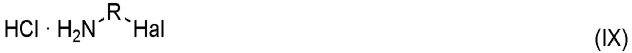 in Ethanol löst und mit einer Base umsetzt, anschließend das Isocyanatsilan der Formel VII 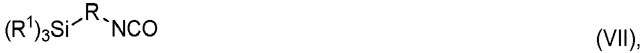 zugibt, anschließend Natriumpolysulfid der Formel V

Na₂Sₓ (V)

zugibt, filtriert und das Lösungsmittel entfernt.

Das Produkt, hergestellt mit dem dritten erfindungsgemäßen Verfahren, kann einen Restgehalt an Hydrochloridsalz des Amins der Formel IX von weniger als 25 mol-%, bevorzugt weniger als 10 mol-%, besonders bevorzugt weniger als 5 mol-%, ganz besonders bevorzugt weniger als 3 mol-%, haben.

Die relativen mol-% des Hydrochloridsalz des Amins der Formel IX in dem Produkt, hergestellt mit dem dritten erfindungsgemäßen Verfahren, werden im ¹³C-NMR durch Integration der C-Atome der -CH₂-NH₂·HCl Gruppe des Hydrochlorid-Salz des Amins der Formel IX gegen die C-Atome der Si-QH₂- Gruppe des harnstoffhaltigen Silans der Formel I bestimmt.
Für die Substanz der Formel IX HCl·H₂N-CH₂-CH₂-Cl wird zum Beispiel das Integral der C-Atome der HCl·H₂N-CH₂-CH₂-Cl Gruppe (δ = 41,25 ppm) oder der HCl·H₂N-CH₂-CH₂-Cl Gruppe (δ = 40,79 ppm) für die Bestimmung der relativen Gehalte verwendet.

Harnstoffhaltige Silane der Formel I hergestellt nach den erfindungsgemäßen Verfahren können durch eine dem Fachmann bekannte ¹H-,¹³C-, oder ²⁹Si-NMR Methode charakterisiert werden.

Der lösliche Anteil der harnstoffhaltigen Silane der Formel I in dem durch die erfindungsgemäße Verfahren erhaltenen Produkte in DMSO-d⁶ oder CDCl₃ wird durch Zugabe eines internen Standards, wie beispielsweise Triphenylphosphinoxid (TPPO), in DMSO-d6 oder in CDCl₃ und eine dem Fachmann bekannte ¹H-NMR Methode ermittelt.

Die harnstoffhaltigen Silane der Formel I können als Haftvermittler zwischen anorganischen Materialien, zum Beispiel Glaskugeln, Glasplittern, Glasoberflächen, Glasfasern, oder oxidischen Füllstoffen, bevorzugt Kieselsäuren wie gefällten Kieselsäuren und Flammkieselsäuren,
und organischen Polymeren, zum Beispiel Duroplasten, Thermoplasten oder Elastomeren, beziehungsweise als Vernetzungsmittel und Oberflächenmodifizierungsmittel für oxidische Oberflächen verwendet werden.

Die harnstoffhaltigen Silane der Formel I können als Kopplungsreagenzien in gefüllten Kautschukmischungen, beispielsweise Reifenlaufflächen, technischen Gummiartikeln oder Schuhsolen, verwendet werden.

Vorteile der erfindungsgemäßen harnstoffhaltigen Silane der Formel I sind verbessertes Verarbeitungsverhalten und Rollwiderstand in Kautschukmischungen.

### Beispiele

### Vergleichsbeispiel 1: Herstellung von [(EtO)₃Si-(CH₂)₃-NH-C(=O)-NH-(CH₂)₂-S-]₂ in Wasser

In einem mit N2 gespülten 1L Doppelmantel-Vierhalskolben mit KPG-Rührer, Rückflusskühler, Innenthermometer und Tropftrichter wird Cystamindihydrochlorid (108,39 g, 0,47 mol, 1,00 eq) vorgelegt und in VE-Wasser (382 mL) gelöst. Mittels Tropftrichter wird 50%ige KOH-Lösung (92,31 g, 0,82 mol, 1,75 eq) bei 15-23°C zudosiert und für 30 min gerührt. Nun wird 3-Isocyanatopropyltriethoxysilan (221,05 g, 0,85 mol, 1,8 eq) so zudosiert, dass eine Innentemperatur von 30°C nicht überschritten wird. Danach wird eine Stunde bei 24°C gerührt. Die weiße Suspension wird über Druck filtriert, mit drei Portionen VE-Wasser (340 mL gesamt) gespült und mit trockenem N₂ für 2 h getrocknet. Der Filterkuchen wird im Rotationsverdampfer für 7 h bei 35°C und 166 mbar, für 10 h bei 35°C und 150 mbar und für 9 h bei 35°C und 100 mbar im N₂-Strom getrocknet. Das Produkt [(EtO)₃Si-(CH₂)₃-NH-C(=O)-NH-(CH₂)₂-S-]₂ ist ein feines, weißes Pulver (246,38 g, 90,7% d.Th.);
¹H-NMR (δₚₚₘ, 500 MHz, DMSO-d6): 0.52 (4H, t), 1.14 (18H, t), 1.42 (4H, m), 2.74 (4H, m), 2.96 (4H, m), 3.29 (4H, m), 3.74 (12H, q), 6.05 (4H, m);
¹³C-NMR (δₚₚₘ, 125 MHz, DMSO-d6): 7.3 (2C), 18.2 (6C), 23.5 (2C), 38.5 (2C), 39.6 (2C), 42.0 (2C), 57.7 (6C) 157.9 (2C).
²⁹Si-NMR (δₚₚₘ, 100 MHz, DMSO-d6): -45.3 (100% Silan);
lösliche Anteile in d6-DMSO unter Verwendung von internem Standard TPPO: 86,0%; Wassergehalt (DIN 51777): 0,7%;
Schmelzbeginn: 97°C;
Restgehalt Isocyanat: 0,08%

### Beispiel 1: Herstellung von (EtO)₃Si-CH₂CH₂CH₂-NH-CO-NH-CH₂CH₂-S₄-CH₂CH₂-NH-CO-NH-CH₂CH₂CH₂-Si(OEt)₃ aus (EtO)₃Si-CH₂CH₂CH₂-NH-CO-NH-CH₂CH₂-S₂-CH₂CH₂-NH-CO-NH-CH₂CH₂CH₂-Si(OEt)₃ und elementarem Schwefel (analog erstem erfindungsgemäßen Verfahren).

In einem trockenen und mit N2 gespülten Dreihalskolben mit Rührer, Rückflusskühler und Innenthermometer wird (EtO)₃Si-CH₂CH₂CH₂-NH-CO-NH-CH₂CH₂-S₂-CH₂CH₂-NH-CO-NH-CH₂CH₂CH₂-Si(OEt)₃ (5,00 g, 7,7 mmol, 1,00 eq) und elementarer Schwefel (0,50 g, 15,5 mol, 2,00 eq) vorgelegt, auf 140°C erhitzt und für 2h gerührt. Nach dem Abkühlen erhält man das Produkt (EtO)₃Si-CH₂CH₂CH₂-NH-CO-NH-CH₂CH₂-S₄-CH₂CH₂-NH-CO-NH-CH₂CH₂CH₂-Si(OEt)₃ (4,16 g, 75,8% d.Th.) als viskoses, rotes Öl, welches später zu einem orangefarbenen Feststoff erstarrt.
¹H-NMR (δₚₚₘ, 500 MHz, CDCl₃): 0.61 (4H, t), 1.20 (18H, t), 1.57 (4H, m), 2.68 (4H · 2S-Anteil, t), 2.93 (4H · S4-Anteil, t), 2.97 (4H · Sx-Anteil, t), 3.13 (4H, m), 3.53 (4H, m), 3.78 (12H, q), 5.1-6.9 (4H, br);
S4-Anteil in der Produktmischung (enthält Sx-Anteil ca. <5%, S4-Anteil überlagert): 85,5 Mol-%, S2-Anteil in der Produktmischung: 14,5 Mol-%;
¹³C-NMR (δₚₚₘ, 125 MHz, CDCl₃): 7.7 (2C), 18.3 (6C), 23.7 (2C), 38.8 (2C), 40.7 (2C), 42.9 (2C), 58.4 (6C), 158.6 (2C).
²⁹Si-NMR (δₚₚₘ, 100 MHz, CDCl₃):-42.9 (5% Si-OH), -45.5 (85% Silan), -53,4 (10% M-Strukturen); lösliche Anteile in CDCl₃ unter Verwendung von internem Standard TPPO: 84,2%; Schmelzbeginn: 170-207°C;

### Beispiel 2: Herstellung von (EtO)₃Si-CH₂CH₂CH₂-NH-CO-NH-CH₂CH₂-S₄-CH₂CH₂-NH-CO-NH-CH₂CH₂CH₂-Si(OEt)₃ aus (EtO)₃Si-CH₂CH₂CH₂-NH₂, OCN-CH₂CH₂-Cl und Na₂S₄ (analog zweitem erfindungsgemäßen Verfahren).

In einem ersten Reaktionsschritt wird 3-Aminopropyltriethoxysilan (73,05 g, 0,33 mol, 1,00 eq) in Pentan (2,5 L) in einem 4 L Dreihalskolben mit KPG-Rührer, Innenthermometer, Tropftrichter und Rückflusskühler vorgelegt und auf-78°C gekühlt. 2-Chlorethylisocyanat (34,82 g, 0,33 mol, 1,00 eq) wird innerhalb von 4,5 h bei -78 bis -70°C zugetropft und danach auf Raumtemperatur erwärmt. Die weiße Suspension wird filtriert, mit Pentan gewaschen und über Nacht mit N₂ getrocknet. Das Zwischenprodukt (EtO)₃Si-CH₂CH₂CH₂-NH-CO-NH-CH₂CH₂-Cl (113,41 g, quantitativ) ist ein weißes, flockiges Pulver.

In einem zweiten Reaktionsschritt wird (EtO)₃Si-CH₂CH₂CH₂-NH-CO-NH-CH₂CH₂-Cl (105,62 g, 0,32 mol, 2,00 eq) in Ethanol (200 mL) in einem 500 mL Dreihalskolben mit Rührer, Rückflusskühler und Innenthermometer vorgelegt. Gemörsertes Natriumpolysulfid (Na₂S₄, 26,59 g, 0,16 mol, 1,00 eq) wird zugegeben und die Mischung zum Rückfluss erhitzt. Nach einer Reaktionszeit von 4,5 h wird auf Raumtemperatur gekühlt und die Suspension filtriert. Das Filtrat wird am Rotationsverdampfer vom Lösungsmittel befreit und am Vakuum getrocknet. Man erhält das Produkt (EtO)₃Si-CH₂CH₂CH₂-NH-CO-NH-CH₂CH₂-S₄-CH₂CH₂-NH-CO-NH-CH₂CH₂CH₂-Si(OEt)₃ (72,35 g, 64,2% d.Th.) als orangenen Feststoff.
¹H-NMR (δₚₚₘ, 500 MHz, d6-Tol): 0.72 (4H, t), 1.21 (18H, t), 1.75 (4H, m), 2.65 (4H · 2S-Anteil, t), 2.89 (4H · S4-Anteil, t), 3.25-3.35 (4H, 2S/4S, m), 3.40-3.60 (4H, 2S/4S, m), 3.81 (12H, q), 5.5-6.0 (4H, br);
S4-Anteil in der Produktmischung (enthält Sx-Anteil ca. <5%): 69,0 Mol-%
S2-Anteil in der Produktmischung: 31,0 Mol-%;
Schmelzbeginn: 78-95°C;

### Beispiel 3: Herstellung von (EtO)₃Si-CH₂CH₂CH₂-NH-CO-NH-CH₂CH₂-S₄-CH₂CH₂-NH-CO-NH-CH₂CH₂CH₂-Si(OEt)₃ aus (EtO)₃Si-CH₂CH₂CH₂-NH₂, OCN-CH₂CH₂-Cl und Na₂S₄ (analog zweitem erfindungsgemäßen Verfahren).

In einem ersten Reaktionsschritt wird 3-Aminopropyltriethoxysilan (154,95 g, 0,70 mol, 1,00 eq) in Ethanol (3,0 L) in einem 4 L Dreihalskolben mit KPG-Rührer, Innenthermometer, Tropftrichter und Rückflusskühler vorgelegt und auf -78°C gekühlt. 2-Chlorethylisocyanat (73,86 g, 0,70 mol, 1,00 eq) wird innerhalb von 1 h bei -78 bis -60°C zugetropft, wobei ein voluminöses Salz ausfällt. Danach wird auf 50°C erhitzt, gemörsertes Natriumpolysulfid (Na₂S₄, 57,62 g, 0,35 mol, 1,00 eq) portionsweise zugegeben und die Mischung zum Rückfluss erhitzt. Nach einer Reaktionszeit von 4,5 h wird auf Raumtemperatur gekühlt und die Suspension filtriert. Das Filtrat wird am Rotationsverdampfer vom Lösungsmittel befreit und am Vakuum getrocknet. Man erhält das Produkt (EtO)₃Si-CH₂CH₂CH₂-NH-CO-NH-CH₂CH₂-S₄-CH₂CH₂-NH-CO-NH-CH₂CH₂CH₂-Si(OEt)₃ (155,05 g, 63,1% d.Th.) als orangenen Feststoff.
¹H-NMR (δₚₚₘ, 500 MHz, d6-Tol): 0.7 (4H, t), 1.21 (18H, t), 1.75 (4H, m), 2.65 (4H · 2S-Anteil, t), 2.89 (4H · S4-Anteil, t), 3.25-3.35 (4H, 2S/4S, m), 3.40-3.60 (4H, 2S/4S, m), 3.81 (12H, q), 5.5-6.0 (4H, br);
S4-Anteil in der Produktmischung (enthält Sx-Anteil ca. <5%): 64,0 Mol-%
S2-Anteil in der Produktmischung: 36,0 Mol-%;
Schmelzbeginn: 78-91°C.

### Beispiel 4: Herstellung von (EtO)₃Si-CH₂CH₂CH₂-NH-CO-NH-CH₂CH₂-S₄-CH₂CH₂-NH-CO-NH-CH₂CH₂CH₂-Si(OEt)₃ aus (EtO)₃Si-CH₂CH₂CH₂-NH₂, OCN-CH₂CH₂-Cl und Na₂S₄ (analog drittem erfindungsgemäßen Verfahren).

In einem ersten Reaktionsschritt wird 2-Chlorethylamin Hydrochlorid (73,86 g, 0,70 mol, 1,00 eq) in Ethanol (3,0 L) in einem 4 L Dreihalskolben mit KPG-Rührer, Innenthermometer, Tropftrichter und Rückflusskühler vorgelegt und auf-78°C gekühlt und Natriumethanolat (226,83 g, 0,70 mol, 1,00 eq, 21% in Ethanol) zugegeben. 3-Isocyanatopropyl(triethoxysilan) (173,15 g, 0,70 mol, 1,00 eq) wird nun innerhalb von 3 h bei -78 bis -70°C zugetropft und danach auf 50°C erwärmt. Trockenes Natriumpolysulfid (Na₂S₄, 57,62 g, 0,35 mol, 0,50 eq) wird in fünf Portionen zugegeben und die Mischung zum Rückfluss erhitzt. Nach einer Reaktionszeit von 4 h wird auf Raumtemperatur gekühlt und die Suspension filtriert. Das Filtrat wird am Rotationsverdampfer vom Lösungsmittel befreit und am Vakuum getrocknet. Man erhält das Produkt (EtO)₃Si-CH₂CH₂CH₂-NH-CO-NH-CH₂CH₂-S₄-CH₂CH₂-NH-CO-NH-CH₂CH₂CH₂-Si(OEt)₃ (214,5 g, quant.) als rotes Öl.

S4-Anteil in der Produktmischung (enthält Sx-Anteil ca. <5%, S4-Anteil überlagert): 85,5 Mol-%, S2-Anteil in der Produktmischung: 14,5 Mol-%.

### Beispiel 5

Die für die Kautschukmischungen verwendete Rezeptur ist in der folgenden Tabelle 1 angegeben. Dabei bedeutet die Einheit phr Gewichtsanteile bezogen auf 100 Teile des eingesetzten Rohkautschuks. Das erfindungsgemäße Silan und das Vergleichssilan werden in 3 verschiedenen Konzentrationen jeweils im isomolaren Verhältnis eingesetzt.

**Tabelle 1**

| Substanz | Menge [phr] | Menge [phr] | Menge [phr] | Menge [phr] | Menge [phr] | Menge [phr] |
|---|---|---|---|---|---|---|
| 1. Stufe | Ref. kaut-schukmischung I enth. Vergl.-beisp.1 | Ref. kautschukmischung II enth. Vergl.-beisp.1 | Ref. kautschukmischung III enth. Vergl.-beisp.1 | Kautschukmischung I, enth. erf. Beisp. 3 | Kautschukmischung II, enth. erf.- Beisp. 3 | Kautschukmischung III, enth. erf.- Beisp. 3 |
| NR TSR^{a} | 10 | 10 | 10 | 10 | 10 | 10 |
| BR^{b} | 18 | 18 | 18 | 18 | 18 | 18 |
| SSBR^{c} | 72 | 72 | 72 | 72 | 72 | 72 |
| Kieselsäure^{d} | 95 | 95 | 95 | 95 | 95 | 95 |
| ZnO | 2,5 | 2,5 | 2,5 | 2,5 | 2,5 | 2,5 |
| Stearinsäure | 2,5 | 2,5 | 2,5 | 2,5 | 2,5 | 2,5 |
| TDAE Öl | 50 | 50 | 50 | 50 | 50 | 50 |
| Ozonschutzwachs | 2 | 2 | 2 | 2 | 2 | 2 |
| 6PPD^{e} | 2 | 2 | 2 | 2 | 2 | 2 |
| Vergl.beisp.1 | 7,9 | 9,3 | 10,7 | | | |
| Beispiel 3 | | | | 8,4 | 9,9 | 11,4 |
| 2. Stufe | | | | | | |
| Batch | | | | | | |
| Stufe 2 | | | | | | |
| DPG^{†} | 2 | 2 | 2 | 2 | 2 | 2 |
| CBS^{g} | 2 | 2 | 2 | 2 | 2 | 2 |
| Schwefel | 2 | 2 | 2 | 2 | 2 | 2 |

| | | | | | | |
|---|---|---|---|---|---|---|
| Verwendete Substanzen: ^{a)} NR TSR: SIR 20 SED, Firma Aneka Bumi Pratama (TSR = Technically Specified Rubber; SIR = Standard Indonesian Rubber) ^{b)} BR: Polybutadien, Europrene Neocis BR 40, Firma Polimeri ^{c)} SSBR: Sprintan^{®} SLR-4601, Firma Styron ^{d)} Kieselsäure: ULTRASIL^{®} VN3 GR, Firma Evonik Industries AG ^{e)} 6PPD: N-(1,3-Dimethylbutyl)-N'-phenyl-p-phenylenediamine ^{f)} DPG: Diphenylguanidin ^{g)} CBS: N-Cyclohexyl-2-benzothiazolsufenamid | | | | | | |

Die Mischungsherstellung erfolgte unter üblichen Bedingungen in zwei Stufen in einem Laborkneter zur Herstellung von Gummimischungen (300 Milliliter bis 3 Liter Volumen), wobei zunächst in der ersten Mischstufe (Grundmischstufe) alle Bestandteile außer dem Vulkanisationssystem (Schwefel und vulkanisationsbeeinflussende Substanzen) für 200 bis 600 Sekunden bei 145 bis 165 °C, Zieltemperaturen von 152 bis 157 °C, vermischt werden. Durch Zugabe des Vulkanisationssystems in der zweiten Stufe (Fertigmischstufe) wird die Fertigmischung erzeugt, wobei für 180 bis 300 Sekunden bei 90 bis 120 °C gemischt wird.

Das allgemeine Verfahren zur Herstellung von Kautschukmischungen und deren Vulkanisate ist in "Rubber Technology Handbook", W. Hofmann, Hanser Verlag 1994 beschrieben.

Die gummitechnische Prüfung erfolgt gemäß den in Tabelle 2 angegebenen Prüfmethoden.

**Tabelle 2:**

| Physikalische Testung | Norm/ Bedingungen |
|---|---|
| Zugversuch am Ring, 23°C | DIN 53504 |
| *Spannungswerte (MPa)* | |
| *Verstärkungsindex* | |
| Rückprallelastizität bei 23°C und 70°C | DIN 53512 |
| (%) | |
| Dynamisch-mechanische Messung bei 55°C | ISO 4664-1 |
| Maximaler Verlustfaktor tan δ (max) | |

Aus sämtlichen Mischungen werden Prüfkörper durch Vulkanisation nach t₉₅ (gemessen am Moving Die Rheometer gemäß ISO 6502 / ASTM D5289-12) unter Druck bei 160°C hergestellt. In der Tabelle 3 sind die gummitechnischen Daten angegeben.

**Tabelle 3:**

| Substanz | Ref. kaut-schukmischung I enth. Vergl.-beisp.1 | Ref. kautschukmischung II enth. Vergl.-beisp.1 | Ref. kautschukmischung III enth. Vergl.-beisp.1 | Kautschukmischung I, enth. erf. Beisp. 3 | Kautschukmischung II, enth. erf. Beisp. 3 | Kautschukmischung III, enth. erf. Beisp. 3 |
|---|---|---|---|---|---|---|
| Vulkanisatergebnisse: | | | | | | |
| Spannungswert 100% [MPa] | 2,1 | 2,3 | 2,5 | 2,7 | 2,8 | 3,0 |
| Spannungswert 300% [MPa] | 6,7 | 7,2 | 7,9 | 8,7 | 9,6 | 10,4 |
| Verstärkungs-index: Spannungswert 300% / 50% [-] | 5,1 | 5,3 | 5,3 | 5,6 | 6,2 | 6,5 |
| Rückprallelast. 23°C [%] | 25,9 | 25,8 | 26,4 | 26,1 | 27,0 | 27,4 |
| Rückprallelast. 70°C [%] | 43,1 | 44,0 | 45,1 | 46,1 | 49,9 | 50,5 |
| Rückprallelast. 70°C - Rückprallelast. 23°C | 17,2 | 18,1 | 18,8 | 20,0 | 23,0 | 23,1 |
| Dynamisch-mechanische Messung bei 55°C tan δ max. bei 55°C | 0,201 | 0,199 | 0,199 | 0,186 | 0,180 | 0,174 |

Die Kautschukmischungen I-III, die das erfindungsgemäße harnstoffhaltige Silan aus Beispiel 3 enthalten, zeigen ein verbessertes Verstärkungsverhalten (höhere Moduli und besserer Verstärkungsindices), verbesserte Rollwiderstandsindikatoren (Rückprallelastizität bei 70°C und tan δ max.). Der Zielkonflikt Rollwiderstand und Nasshaftung wird mit dem erfindungsgemäßen harnstoffhaltigem Silan besser gelöst (Rückprallelastizität 70°C - Rückprallelastizität 23°C, Rückprallelastizität bei 70°C und tan δ max.) gegenüber dem jeweiligen isomolar eingesetzten Vergleichsbeispiel.

## Patentansprüche

1. Harnstoffhaltiges Silan der Formel I 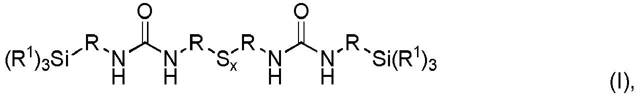 wobei R¹ gleich oder verschieden sind und C1-C10-Alkoxygruppen, C2-C10- cyklische Dialkoxy-Gruppen, Phenoxygruppe, C4-C10-Cycloalkoxygruppen, C6-C18-Arylgruppen, C1-C10-Alkylgruppen, C2-C20-Alkenylgruppe, C7-C20-Aralkylgruppe oder Halogen, und R gleich oder verschieden sind und eine verzweigte oder unverzweigte, gesättigte oder ungesättigte, aliphatische, aromatische oder gemischt aliphatische/aromatische zweibindige C₁-C₃₀-Kohlenwasserstoffgruppe sind und x eine ganze Zahl von 3 bis 8 ist.

2. Harnstoffhaltiges Silan gemäß Anspruch 1, **dadurch gekennzeichnet, dass** das harnstoffhaltige Silan der Formel I
((EtO)₃Si-CH₂-NH-CO-NH-CH₂-S_{x/2})₂,
((EtO)₃Si-CH₂CH₂-NH-CO-NH-CH₂-S_{x/2})₂,
((EtO)₃Si-CH₂-NH-CO-NH-CH₂CH₂-S_{x/2})₂,
((EtO)₃Si-CH₂CH₂-NH-CO-NH-CH₂CH₂-S_{x/2})₂,
((EtO)₃Si-CH₂CH₂CH₂-NH-CO-NH-CH₂-S_{x/2})₂,
((EtO)₃Si-CH₂CH₂CH₂-NH-CO-NH-CH₂CH₂-S_{x/2})₂,
((EtO)₃Si-CH₂-NH-CO-NH-CH₂CH₂CH₂-S_{x/2})₂,
((EtO)₃Si-CH₂CH₂-NH-CO-NH-CH₂CH₂CH₂-S_{x/2})₂,
((EtO)₃Si-CH₂CH₂CH₂-NH-CO-NH-CH₂CH₂CH₂-S_{x/2})₂,
((MeO)₃Si-CH₂-NH-CO-NH-CH₂-S_{x/2})₂,
((MeO)₃Si-CH₂CH₂-NH-CO-NH-CH₂-S_{x/2})₂,
((MeO)₃Si-CH₂-NH-CO-NH-CH₂CH₂-S_{x/2})₂,
((MeO)₃Si-CH₂CH₂-NH-CO-NH-CH₂CH₂-S_{x/2})₂,
((MeO)₃Si-CH₂CH₂CH₂-NH-CO-NH-CH₂-S_{x/2})₂,
((MeO)₃Si-CH₂CH₂CH₂-NH-CO-NH-CH₂CH₂-S_{x/2})₂,
((MeO)₃Si-CH₂-NH-CO-NH-CH₂CH₂CH₂-S_{x/2})₂,
((MeO)₃Si-CH₂CH₂-NH-CO-NH-CH₂CH₂CH₂-S_{x/2})₂ oder
((MeO)₃Si-CH₂CH₂CH₂-NH-CO-NH-CH₂CH₂CH₂S_{x/2})₂ ist.

3. Harnstoffhaltiges Silan gemäß Anspruch 1, **dadurch gekennzeichnet, dass** das harnstoffhaltiges Silan der Formel I
(EtO)₃Si-CH₂CH₂CH₂-NH-CO-NH-CH₂CH₂-S₄-CH₂CH₂-NH-CO-NH-CH₂CH₂CH₂-Si(OEt)₃
ist.

4. Verfahren zur Herstellung von harnstoffhaltigem Silan der Formel I gemäß Anspruch 1, **dadurch gekennzeichnet, dass** man harnstoffhaltiges Disulfidsilan der Formel II 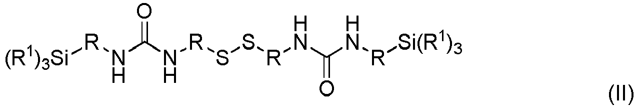 mit Schwefel umsetzt, wobei R und R¹ die in Anspruch 1 genannten Bedeutungen haben.

5. Verfahren zur Herstellung von harnstoffhaltigem Silan der Formel I gemäß Anspruch 4, **dadurch gekennzeichnet, dass** man die Umsetzung unter Schutzgasatmosphäre durchgeführt.

6. Verfahren zur Herstellung von harnstoffhaltigem Silan der Formel I gemäß Anspruch 4, **dadurch gekennzeichnet, dass** man die Umsetzung unter Normaldruck durchführt.

7. Verfahren zur Herstellung von harnstoffhaltigem Silan der Formel I gemäß Anspruch 4, **dadurch gekennzeichnet, dass** man die Umsetzung zwischen 30°C - 180°C durchführt.

8. Verfahren zur Herstellung von harnstoffhaltigem Silan der Formel I gemäß Anspruch 4, **dadurch gekennzeichnet, dass** die Umsetzung ohne Lösungsmittel durchgeführt wird.

9. Verfahren zur Herstellung von harnstoffhaltigem Silan der Formel I gemäß Anspruch 1, **dadurch gekennzeichnet, dass** man in einem ersten Schritt ein Aminosilan der Formel III 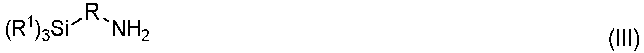 mit einem Isocyanat der Formel IV 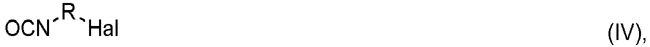
wobei R und R¹ die in Anspruch 1 genannte Bedeutungen haben und Hal gleich F, Cl, Br oder I, ist,
umsetzt
und in einem zweiten Schritt das Produkt aus dem ersten Verfahrensschritt mit Natriumpolysulfid der Formel (V)
Na₂Sₓ (V)
wobei x die in Anspruch 1 gennante Bedeutung hat,
umsetzt.

10. Verfahren zur Herstellung von harnstoffhaltigem Silan der Formel I gemäß Anspruch 9,
**dadurch gekennzeichnet, dass** man den ersten Schritt unter Schutzgasatmosphäre durchführt.

11. Verfahren zur Herstellung von harnstoffhaltigem Silan der Formel I gemäß der Ansprüche 9 oder 10, **dadurch gekennzeichnet, dass** man den zweiten Schritt unter Schutzgasatmosphäre durchführt.

12. Verfahren zur Herstellung von harnstoffhaltigem Silan der Formel I gemäß Anspruch 9,
**dadurch gekennzeichnet, dass** man den ersten Schritt bei Temperaturen zwischen -78°C und 100°C durchführt.

13. Verfahren zur Herstellung von harnstoffhaltigem Silan der Formel I gemäß den Ansprüchen 9 oder 12, **dadurch gekennzeichnet, dass** man den zweiten Schritt bei Temperaturen zwischen 20°C und 150°C durchführt.

14. Verfahren zur Herstellung von harnstoffhaltigem Silan der Formel I gemäß Anspruch 9,
**dadurch gekennzeichnet, dass** man in dem ersten Schritt Ethanol als Lösungsmittel verwendet.

15. Verfahren zur Herstellung von harnstoffhaltigem Silan der Formel I gemäß den Ansprüchen 9 oder 14, **dadurch gekennzeichnet, dass** man in dem zweiten Schritt Ethanol als Lösungsmittel verwendet.

16. Verfahren zur Herstellung von harnstoffhaltigem Silan der Formel I gemäß den Ansprüchen 14 oder 15, **dadurch gekennzeichnet, dass** man den Alkohol nach dem ersten oder zweiten Schritt abdestilliert.

17. Verfahren zur Herstellung von harnstoffhaltigem Silan der Formel I gemäß Anspruch 1, **dadurch gekennzeichnet, dass** man in einem ersten Schritt ein Isocyanatosilan der Formel VII 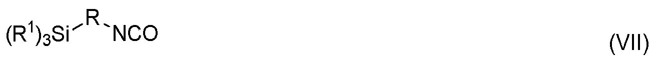 mit einem Amin der Formel VIII 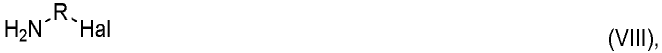
wobei R und R¹ die in Anspruch 1 genannte Bedeutungen haben und Hal gleich F, Cl, Br oder I, ist,
umsetzt
und in einem zweiten Schritt das Produkt aus dem ersten Verfahrensschritt mit Natriumpolysulfid der Formel V
Na₂Sₓ (V)
wobei x die in Anspruch 1 genannte Bedeutung hat,
umsetzt.

18. Verfahren zur Herstellung von harnstoffhaltigem Silan der Formel I gemäß Anspruch 17, **dadurch gekennzeichnet, dass** das Amin der Formel VIII vor der Umsetzung mit den Isocyanatsilan der Formel VII aus dem Hydrochlorid-Salz des Diamin der Formel IX
Cl⁻⁺H₃N-R-S-S-R-NH₃⁺Cl⁻ IX
durch Zugabe einer Base hergestellt wird.

19. Verfahren zur Herstellung von harnstoffhaltigem Silan der Formel I gemäß Anspruch 18, **dadurch gekennzeichnet, dass** man als Base NaOEt einsetzt.

20. Verfahren zur Herstellung von harnstoffhaltigem Silan der Formel I gemäß den Ansprüchen 17 oder 18, **dadurch gekennzeichnet, dass** man in dem ersten Schritt Ethanol als Lösungsmittel verwendet.

21. Verfahren zur Herstellung von harnstoffhaltigem Silan der Formel I gemäß den Ansprüchen 17 oder 18, **dadurch gekennzeichnet, dass** man in dem zweiten Schritt Ethanol als Lösungsmittel verwendet.

22. Verfahren zur Herstellung von harnstoffhaltigem Silan der Formel I gemäß den Ansprüchen 20 oder 21, **dadurch gekennzeichnet, dass** man den Alkohol nach dem ersten oder zweiten Schritt abdestilliert.

23. Verfahren zur Herstellung von harnstoffhaltigem Silan der Formel I gemäß den Ansprüchen 17 oder 22, **dadurch gekennzeichnet, dass** man das Produkt trocknet.

## Claims

1. Urea-containing silane of the formula I 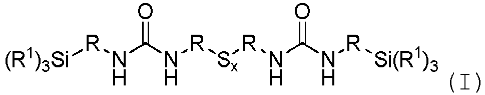 where R¹ are the same or different and are C1-C10 alkoxy groups, C2-C10 cyclic dialkoxy groups, phenoxy group, C4-C10 cycloalkoxy groups, C6-C18 aryl groups, C1-C10 alkyl groups, C2-C20 alkenyl group, C7-C20 aralkyl group or halogen, and R are the same or different and are a branched or unbranched, saturated or unsaturated, aliphatic, aromatic or mixed aliphatic/aromatic divalent C₁-C₃₀ hydrocarbon group and x is an integer from 3 to 8.

2. Urea-containing silane according to Claim 1, **characterized in that** the urea-containing silane is of the formula I
((EtO)₃Si-CH₂-NH-CO-NH-CH₂-S_{x/2})₂,
((EtO)₃Si-CH₂CH₂-NH-CO-NH-CH₂-S_{x/2})₂,
((EtO)₃Si-CH₂-NH-CO-NH-CH₂CH₂-S_{x/2})₂,
((EtO)₃Si-CH₂CH₂-NH-CO-NH-CH₂CH₂-S_{x/2})₂,
((EtO)₃Si-CH₂CH₂CH₂-NH-CO-NH-CH₂-S_{x/2})₂,
((EtO)₃Si-CH₂CH₂CH₂-NH-CO-NH-CH₂CH₂-S_{x/2})₂,
((EtO)₃Si-CH₂-NH-CO-NH-CH₂CH₂CH₂-S_{x/2})₂,
((EtO)₃Si-CH₂CH₂-NH-CO-NH-CH₂CH₂CH₂-S_{x/2})₂,
((EtO)₃Si-CH₂CH₂CH₂-NH-CO-NH-CH₂CH₂CH₂-S_{x/2})₂,
((MeO)₃Si-CH₂-NH-CO-NH-CH₂-S_{x/2})₂,
((MeO)₃Si-CH₂CH₂-NH-CO-NH-CH₂-S_{x/2})₂,
((MeO)₃Si-CH₂-NH-CO-NH-CH₂CH₂-S_{x/2})₂,
((MeO)₃Si-CH₂CH₂-NH-CO-NH-CH₂CH₂-S_{x/2})₂,
((MeO)₃Si-CH₂CH₂CH₂-NH-CO-NH-CH₂-S_{x/2})₂,
((MeO)₃Si-CH₂CH₂CH₂-NH-CO-NH-CH₂CH₂-S_{x/2})₂,
((MeO)₃Si-CH₂-NH-CO-NH-CH₂CH₂CH₂-S_{x/2})₂, ((MeO)₃Si-CH₂CH₂-NH-CO-NH-CH₂CH₂CH₂-S_{x/2})₂ or
((MeO)₃Si-CH₂CH₂CH₂-NH-CO-NH-CH₂CH₂CH₂-S_{x/2})₂.

3. Urea-containing silane according to Claim 1, **characterized in that** the urea-containing silane is of the formula I
(EtO)₃Si-CH₂CH₂CH₂-NH-CO-NH-CH₂CH₂-S₄-CH₂CH₂-NH-CO-NH-CH₂CH₂CH₂-Si(OEt)₃.

4. Process for preparing urea-containing silane of the formula I according to Claim 1, **characterized in that** urea-containing disulphide silane of the formula II 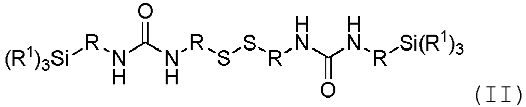 is reacted with sulphur, where R and R¹ are each as defined in Claim 1.

5. Process for preparing urea-containing silane of the formula I according to Claim 4, **characterized in that** the reaction is conducted under a protective gas atmosphere.

6. Process for preparing urea-containing silane of the formula I according to Claim 4, **characterized in that** the reaction is conducted under standard pressure.

7. Process for preparing urea-containing silane of the formula I according to Claim 4, **characterized in that** the reaction is conducted between 30°C-180°C.

8. Process for preparing urea-containing silane of the formula I according to Claim 4, **characterized in that** the reaction is conducted without solvent.

9. Process for preparing urea-containing silane of the formula I according to Claim 1, **characterized in that**, in a first step, an aminosilane of the formula III 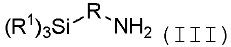 is reacted with an isocyanate of the formula IV 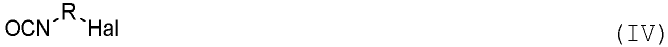
where R and R¹ are each as defined in Claim 1 and Hal is F, Cl, Br or I,
and, in a second step, the product from the first process step is reacted with sodium polysulphide of the formula (V)
Na₂Sₓ (V)
where x is as defined in Claim 1.

10. Process for preparing urea-containing silane of the formula I according to Claim 9, **characterized in that** the first step is conducted under a protective gas atmosphere.

11. Process for preparing urea-containing silane of the formula I according to either of Claims 9 and 10, **characterized in that** the second step is conducted under a protective gas atmosphere.

12. Process for preparing urea-containing silane of the formula I according to Claim 9, **characterized in that** the first step is conducted at temperatures between -78°C and 100°C.

13. Process for preparing urea-containing silane of the formula I according to Claim 9 or 12, **characterized in that** the second step is conducted at temperatures between 20°C and 150°C.

14. Process for preparing urea-containing silane of the formula I according to Claim 9, **characterized in that** ethanol is used as solvent in the first step.

15. Process for preparing urea-containing silane of the formula I according to Claim 9 or 14, **characterized in that** ethanol is used as solvent in the second step.

16. Process for preparing urea-containing silane of the formula I according to Claim 14 or 15, **characterized in that** the alcohol is distilled off after the first or second step.

17. Process for preparing urea-containing silane of the formula I according to Claim 1, **characterized in that**, in a first step, an isocyanatosilane of the formula VII 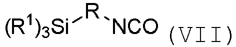 is reacted with an amine of the formula VIII 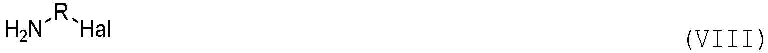 where R and R¹ are each as defined in Claim 1 and Hal is F, Cl, Br or I,
and, in a second step, the product from the first process step is reacted with sodium polysulphide of the formula V
Na₂Sₓ (V)
where x is as defined in Claim 1.

18. Process for preparing urea-containing silane of the formula I according to Claim 17, **characterized in that** the amine of the formula VIII, prior to reaction with the isocyanatosilane of the formula VII, is prepared from the hydrochloride salt of the diamine of the formula IX
Cl^{- +}H₃N-R-S-S-R-NH₃⁺Cl⁻ IX
by addition of a base.

19. Process for preparing urea-containing silane of the formula I according to Claim 18, **characterized in that** the base used is NaOEt.

20. Process for preparing urea-containing silane of the formula I according to Claim 17 or 18, **characterized in that** ethanol is used as solvent in the first step.

21. Process for preparing urea-containing silane of the formula I according to Claim 17 or 18, **characterized in that** ethanol is used as solvent in the second step.

22. Process for preparing urea-containing silane of the formula I according to Claim 20 or 21, **characterized in that** the alcohol is distilled off after the first or second step.

23. Process for preparing urea-containing silane of the formula I according to Claim 17 or 22, **characterized in that** the product is dried.

## Revendications

1. Silane, contenant de l'urée, de formule I 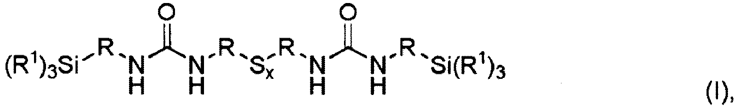 dans laquelle les radicaux R¹ sont identiques ou différents et représentent des groupes C₁-C₁₀-alcoxy, des groupes C₂-C₁₀-dialcoxy cycliques, le groupe phénoxy, des groupes C₄-C₁₀-cycloalcoxy, des groupes C₆-C₁₈-aryle, des groupes C₁-C₁₀-alkyle, des groupes C₂-C₂₀-alcényle, des groupes C₇-C₂₀-aralkyle ou halogène, et les radicaux R sont identiques ou différents et représentent un groupe hydrocarboné en C₁-C₃₀ divalent, ramifié ou non ramifié, saturé ou insaturé, aliphatique, aromatique ou aliphatique/aromatique mixte et x vaut un nombre entier de 3 à 8.

2. Silane, contenant de l'urée, selon la revendication 1, **caractérisé en ce que** le silane, contenant de l'urée, de formule I est
((EtO)₃Si-CH₂-NH-CO-NH-CH₂-S_{x/2})₂,
((EtO)₃Si-CH₂CH₂-NH-CO-NH-CH₂-S_{x/2})₂,
((EtO)₃Si-CH₂-NH-CO-NH-CH₂CH₂-S_{x/2})₂,
((EtO)₃Si-CH₂CH₂-NH-CO-NH-CH₂CH₂-S_{x/2})₂,
((EtO)₃Si-CH₂CH₂CH₂-NH-CO-NH-CH₂-S_{x/2})₂,
((EtO)₃Si-CH₂CH₂CH₂-NH-CO-NH-CH₂CH₂-S_{x/2})₂,
((EtO)₃Si-CH₂-NH-CO-NH-CH₂CH₂CH₂-S_{x/2})₂,
((EtO)₃Si-CH₂CH₂-NH-CO-NH-CH₂CH₂CH₂-S_{x/2})₂,
((EtO)₃Si-CH₂CH₂CH₂-NH-CO-NH-CH₂CH₂CH₂-S_{x/2})₂,
((MeO)₃Si-CH₂-NH-CO-NH-CH₂-S_{x/2})₂,
((MeO)₃Si-CH₂CH₂-NH-CO-NH-CH₂-S_{x/2})₂,
((MeO)₃Si-CH₂-NH-CO-NH-CH₂CH₂-S_{x/2})₂,
((MeO)₃Si-CH₂CH₂-NH-CO-NH-CH₂CH₂-S_{x/2})₂,
((MeO)₃Si-CH₂CH₂CH₂-NH-CO-NH-CH₂-S_{x/2})₂,
((MeO)₃Si-CH₂CH₂CH₂-NH-CO-NH-CH₂CH₂-S_{x/2})₂,
((MeO)₃Si-CH₂-NH-CO-NH-CH₂CH₂CH₂-S_{x/2})₂, ((MeO)₃Si-CH₂CH₂-NH-CO-NH-CH₂CH₂CH₂-S_{x/2})₂ ou ((MeO)₃Si-CH₂CH₂CH₂-NH-CO-NH-CH₂CH₂CH₂-S_{x/2})₂.

3. Silane, contenant de l'urée, selon la revendication 1, **caractérisé en ce que** le silane, contenant de l'urée, de formule I est
(EtO)₃Si-CH₂CH₂CH₂-NH-CO-NH-CH₂CH₂-S₄-CH₂CH₂-NH-CO-NH-CH₂CH₂CH₂-Si(OEt)₃.

4. Procédé pour la préparation de silane, contenant de l'urée, de formule I selon la revendication 1, **caractérisé en ce qu'**on transforme du dithiosilane, contenant de l'urée, de formule II 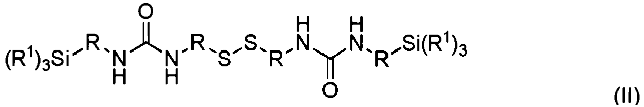 avec du soufre, R et R¹ présentant les significations mentionnées dans la revendication 1.

5. Procédé pour la préparation de silane, contenant de l'urée, de formule I selon la revendication 4, **caractérisé en ce qu'**on réalise la transformation sous une atmosphère de gaz protecteur.

6. Procédé pour la préparation de silane, contenant de l'urée, de formule I selon la revendication 4, **caractérisé en ce qu'**on réalise la transformation sous pression normale.

7. Procédé pour la préparation de silane, contenant de l'urée, de formule I selon la revendication 4, **caractérisé en ce qu'**on réalise la transformation entre 30°C-180°C.

8. Procédé pour la préparation de silane, contenant de l'urée, de formule I selon la revendication 4, **caractérisé en ce qu'**on réalise la transformation sans solvant.

9. Procédé pour la préparation de silane, contenant de l'urée, de formule I selon la revendication 1, **caractérisé en ce qu'**on transforme, dans une première étape, un aminosilane de formule III 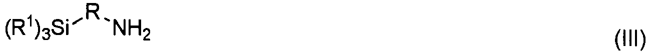 avec un isocyanate de formule IV 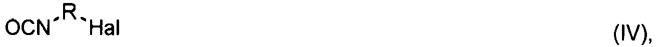 dans laquelle R et R¹ présentent les significations mentionnées dans la revendication 1 et Hal représente F, Cl, Br ou I et, dans une deuxième étape, on transforme le produit de la première étape de procédé avec du polysulfure de sodium de formule (V)
Na₂Sₓ (V)
dans laquelle x présente la signification mentionnée dans la revendication 1.

10. Procédé pour la préparation de silane, contenant de l'urée, de formule I selon la revendication 9, **caractérisé en ce qu'**on réalise la première étape sous une atmosphère de gaz protecteur.

11. Procédé pour la préparation de silane, contenant de l'urée, de formule I selon les revendications 9 ou 10, **caractérisé en ce qu'**on réalise la deuxième étape sous une atmosphère de gaz protecteur.

12. Procédé pour la préparation de silane, contenant de l'urée, de formule I selon la revendication 9, **caractérisé en ce qu'**on réalise la première étape à des températures entre -78 et 100°C.

13. Procédé pour la préparation de silane, contenant de l'urée, de formule I selon les revendications 9 ou 12, **caractérisé en ce qu'**on réalise la deuxième étape à des températures entre 20 et 150°C.

14. Procédé pour la préparation de silane, contenant de l'urée, de formule I selon la revendication 9, **caractérisé en ce qu'**on utilise dans la première étape de l'éthanol comme solvant.

15. Procédé pour la préparation de silane, contenant de l'urée, de formule I selon les revendications 9 ou 14, **caractérisé en ce qu'**on utilise dans la deuxième étape de l'éthanol comme solvant.

16. Procédé pour la préparation de silane, contenant de l'urée, de formule I selon les revendications 14 ou 15, **caractérisé en ce qu'**on élimine, par distillation, l'alcool après la première ou la deuxième étape.

17. Procédé pour la préparation de silane, contenant de l'urée, de formule I selon la revendication 1, **caractérisé en ce qu'**on transforme, dans une première étape, un isocyanatosilane de formule VII 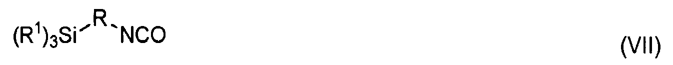 avec une amine de formule VIII 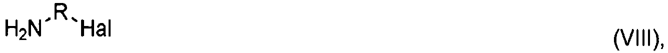 dans lesquelles R et R¹ présentent les significations mentionnées dans la revendication 1 et Hal représente F, Cl, Br ou I et, dans une deuxième étape, on transforme le produit de la première étape de procédé avec du polysulfure de sodium de formule V
Na₂Sₓ (V)
dans laquelle x présente la signification mentionnée dans la revendication 1.

18. Procédé pour la préparation de silane, contenant de l'urée, de formule I selon la revendication 17, **caractérisé en ce que** l'amine de formule VIII est préparée avant la transformation avec l'isocyanatosilane de formule VII à partir du sel de chlorhydrate de la diamine de formule IX
Cl⁻⁺H₃N-R-S-S-R-NH₃⁺Cl⁻ IX
par addition d'une base.

19. Procédé pour la préparation de silane, contenant de l'urée, de formule I selon la revendication 18, **caractérisé en ce qu'**on utilise, comme base, du NaOEt.

20. Procédé pour la préparation de silane, contenant de l'urée, de formule I selon les revendications 17 ou 18, **caractérisé en ce qu'**on utilise dans la première étape de l'éthanol comme solvant.

21. Procédé pour la préparation de silane, contenant de l'urée, de formule I selon les revendications 17 ou 18, **caractérisé en ce qu'**on utilise dans la deuxième étape de l'éthanol comme solvant.

22. Procédé pour la préparation de silane, contenant de l'urée, de formule I selon les revendications 20 ou 21, **caractérisé en ce qu'**on élimine, par distillation, l'alcool après la première ou la deuxième étape.

23. Procédé pour la préparation de silane, contenant de l'urée, de formule I selon les revendications 17 ou 22, **caractérisé en ce qu'**on sèche le produit.
